## Europäisches Patentamt
## European Patent Office
## Office européen des brevets

(11) Veröffentlichungsnummer: **0 229 234**
**A1**

(12) # EUROPÄISCHE PATENTANMELDUNG

(21) Anmeldenummer: **86114361.8**

(22) Anmeldetag: **16.10.86**

(51) Int. Cl.³: **C 12 Q 1/56**
**C 12 Q 1/38**
**//G01N33/68**

(30) Priorität: **16.10.85 DE 3536903**

(43) Veröffentlichungstag der Anmeldung:
**22.07.87 Patentblatt 87/30**

(84) Benannte Vertragsstaaten:
**AT BE CH DE ES FR GB GR IT LI LU NL SE**

(71) Anmelder: **BOEHRINGER MANNHEIM GMBH**
**Patentabteilung, Abt. E Sandhofer Strasse 112-132**
**Postfach 31 01 20**
**D-6800 Mannheim 31 Waldhof(DE)**

(72) Erfinder: **Bartl, Knut, Dr. rer. nat.**
**Am Westend 6**
**D-8121 Wilzhofen(DE)**

(72) Erfinder: **Dessauer, Andreas, Dr. rer. nat.**
**Herre-Strasse 1**
**D-8132 Tutzing(DE)**

(72) Erfinder: **Lill, Helmut, Dr. rer. nat.**
**Zugspitzstrasse 24**
**D-8121 Wielenbach(DE)**

(74) Vertreter: **Weickmann, Heinrich, Dipl.-Ing. et al,**
**Patentanwälte Dipl.-Ing. H.Weickmann Dipl.-Phys.Dr.**
**K.Fincke Dipl.-Ing. F.A.Weickmann Dipl.-Chem. B. Huber**
**Dr.-Ing. H. Liska Dipl.-Phys.Dr. J. Prechtel Postfach 860820**
**D-8000 München 86(DE)**

(54) **Verfahren zur photometrischen Bestimmung von Protein C.**

(57) Zur photometrischen Bestimmung von Protein C, insbesondere im Plasma, wird die das zu bestimmende Protein C enthaltende Probe mit Thrombin unter Bildung von aktiviertem Protein C inkubiert, danuch überschüssiger Thrombininhibitor, wie z. B. Antithrombin III, zugesetzt und anschließend die Abnahme der durch Gerinnungsfaktoren vermittelten Bildung von Thrombin aus Prothrombin mittels eines chromogenen Thrombinsubstrates wie z. B. H-D-Phe-Pip-Arg-pNA oder Tos-Gly-Pro-Arg-pNA, bestimmt.

MESSWERTPAARE
PC-BEST. UEBER APTT ( MANUELL )
X-ACHSE : % PC
Y-ACHSE : APTT (SEC)

FIG .1

N = 7
REGRESSION VON Y AUF X : Y = 57.883 + .706 ⋅ X
REGRESSION VON X AUF Y : Y = 56.549 + .728 ⋅ X
STAND. HAUPTKOMP. ANALYSE : Y = 57.221 + .717 ⋅ X
KORRELATIONSKOEFFIZIENT : 0.984

EP 0 229 234 A1

B e s c h r e i b u n g

Die Erfindung betrifft ein Verfahren zur photometrischen Bestimmung von Protein C, im besonderen im Plasma.

Protein C ist ein zweikettiges, Vitamin K abhängiges Glykoprotein im Plasma. Seine Synthese erfolgt in der Leber. Dabei wird zunächst eine gerinnungsphysiologisch indifferente Vorstufe (Decarboxy-Protein C) gebildet. Carboxylierung von $\gamma$-Glutaminsäureresten im Protein durch eine Vitamin K-abhängige Carboxylase führt zum Protein C. Protein C selbst ist ein Proenzym und wird durch Thrombin in aktiviertes Protein C überführt. Letzteres wirkt durch die Inhibierung der aktivierten Gerinnungsfaktoren V und VIII als ein Antikoagulans. Erniedrigte Protein-C-Spiegel wurden bei Patienten mit Lebererkrankungen, Verbrauchs-Coagulopathie (DIC) und nach Warfarintherapie beschrieben. Ein angeborener Mangel an Protein C führt zu venösen thromboembolischen Risiken. Protein C spielt daher eine wichtige Rolle sowohl bei der physiologischen Hämostasis, als auch in vielen Krankheiten, im besonderen bei der Thrombosis. Infolgedessen besteht auch ein Bedarf nach einer einfachen, insbesondere photometrischen Bestimmungsmethode für Protein C. Ein spezifisches chromogenes Protein-C-Substrat, das seine direkte photometrische Bestimmung erlauben würde, ist jedoch nicht bekannt.

Bei dem einzigen, bislang im Handel erhältlichen Testverfahren wird die Menge an Protein C im Serum durch enzymmarkierte Antikörper bestimmt. Dieses Verfahren hat jedoch den Nachteil, daß die zur Bestimmung eingesetzten Antikörper auch mit dem oben erwähnten Decarboxyprotein C reagieren. Da die Serumkonzentration an
Decarboxyprotein während einer Behandlung mit Antikoagulantien häufig stark ansteigt, birgt dieses Verfahren
eine große Fehlerquelle, die unter Umständen zu einer
falschen oder unzureichenden Therapie führen kann.
R. B. Francis und M. J. Patch (Thrombosis Research 32,
605-613, 1983) lehren ein Verfahren zur Bestimmung von
aktiviertem Protein C im Humanplasma durch Bestimmung
der Partialthromboplastinzeit (PTT-Test). Dabei wird
das Protein C durch Zusatz von Thrombin aktiviert und
danach letzteres durch einen Unterschuß an Antithrombin
III und Heparin inhibiert. Heparin wiederum wird durch
eine jeweils neu zu bestimmende exakte Menge von Protaminsulfat neutralisiert. Danach kann Protein C über
die partielle Thromboplastinzeit bestimmt werden.

Der Nachteil bei der Verwendung immunologischer Bestimmungsverfahren im Plasma für Protein C liegt darin, daß
sie keine Information über die biologische Aktivität
der Protein-C-Moleküle liefern. Die Präsenz von abnormalem Protein C mit stark reduzierter biologischer
Aktivität (genetische Variante) kann mit solchen Verfahren nicht gefunden werden.

R. M. Bertina et al (Thromb. Haemostas 51, (1) 1-5,
(1984)) lehren ein spektrophotometrisches Verfahren zur
Bestimmung der Protein C-Aktivität. Dieses Verfahren
umfaßt drei unabhängige Schritte:

1.    Isolierung des Protein C mit Hilfe einer Al(OH)$_3$-
      Adsorption.

2.  Aktivierung des vom Plasma abgetrennten Protein C
    mit Thrombin, sowie nachfolgende Inhibierung des
    letzteren durch äquimolare Mengen Antithrombin III
    und Heparin.

3.  Messen der proteolytischen Aktivität von isoliertem aktiviertem Protein C mit einem chromogenen
    Substrat ($S_{2366}$=H pyro-Glu-Pro-Arg-pNA).

Dieses Verfahren ist unbefriedigend bezüglich der
Spezifität für Protein C, da dieses Substrat auch von
anderen Gerinnungsproteasen gespalten wird. Es kann
somit zu falsch positiven Ergebnissen kommen.

Ziel der Erfindung ist es daher, einen einfachen und
zuverlässigen optischen Test zur Bestimmung von aktivem
Protein C zur Verfügung zu stellen.

Aktiviertes Protein C inaktiviert die Gerinnungsfaktoren
Va und VIIIa, welche wiederum die Bildung von Thrombin
aus dessen nichtaktiver Vorstufe, dem Prothrombin aktivieren. Man konnte daher daran denken, aktives Protein
C über die Bildung bzw. die Hemmung der Thrombinbildung
aus Prothrombin zu bestimmen. Da aber Protein C zuerst
durch Thrombin aktiviert werden muß, anschließend aber
die Aktivität von aus Prothrombin gebildeten Thrombin
bestimmt werden soll, müßte das zuvor für die Aktivierung des Protein zugesetzte Thrombin vollständig entfernt oder inaktiviert werden. Bei dieser Inaktivierung
müßte der Inhibitor, um das beim Bestimmungsverfahren
gebildete Thrombin nicht ebenfalls zu inhibieren, so
exakt dosiert werden, daß die Durchführung eines Bestimmungsverfahren für Protein C nach dem oben erwähnten
Muster für die Routinepraxis undurchführbar wäre.

Es wurde nun überraschenderweise gefunden, daß das zugesetzte Thrombin durch einen Überschuß an Antithrombin III inaktiviert werden kann, ohne daß Antithrombin III die Meßreaktionen verfälscht.

Das erfindungsgemäße Verfahren zur photometrischen Bestimmung von Protein C, insbesondere im Plasma, ist daher dadurch gekennzeichnet, daß die das zu bestimmende Protein C enthaltende Probe mit Thrombin unter Bildung von aktiviertem Protein C inkubiert wird, danach überschüssiger Thrombininhibitor zugesetzt wird und anschließend die Abnahme der durch Gerinnungsfaktoren vermittelten Bildung von Thrombin aus Prothrombin mittels eines chromogenen Thrombinsubstrates bestimmt wird.

Als Thrombin-Inhibitor kann einer der bekannten Thrombin-Inhibitoren wie Antithrombin III (AT III), gegebenenfalls unter Zusatz von Heparin oder heparinartigen Substanzen verwendet werden. Bevorzugt wird im Rahmen der Erfindung als Thrombininhibitor AT III verwendet. Geeignete Mengen hängen zwar grundsätzlich ab von der vorhandenen Thrombinmenge, da ja ein Überschuß an Inhibitor verwendet werden muß. Im allgemeinen werden jedoch AT III-Mengen zwischen 0,5 und 100 U/ml, vorzugsweise 1 bis 20 U/ml Testvolumen angewendet. Der Inhibitorüberschuß sollte im allgemeinen wenigstens 10 %, vorzugsweise mehr als 20 %, bezogen auf die Thrombinaktivität, betragen. Werden beispielsweise zur Protein-C-Aktivierung 0,5 U Thrombin eingesetzt, so verwendet man zweckmäßig nach der Aktivierung 0,7 U oder mehr an Thrombininhibitor, wie insbesondere AT III.

Da aktiviertes Protein C (APC) die Gerinnungsfaktoren V und VIII proteolytisch inaktiviert, wird beim erfindungsgemäßen Verfahren vorzugsweise das Gerinnungssystem durch Zusatz von Aktivatoren für Gerinnungsfaktoren oder/und von Gerinnungsfaktoren selbst derart modifiziert, daß sich die Inaktivierung der Faktoren V bzw. VIII in einer möglichst ausgeprägten Verringerung der Thrombinbildung auswirkt. Gemäß einer ersten Ausführungsform des erfindungsgemäßen Verfahrens wird dies zweckmäßig dadurch erreicht, daß man einen Aktivator für Faktor XII, beispielsweise Ellagsäure unter Zusatz von Kephalin, zusetzt. Gemäß einer zweiten Ausführungsform dieser bevorzugten Variante gibt man einen Aktivator für Faktor VII, z. B. Thromboplastin, und Faktor V zu. Gemäß einer dritten Ausführungsform setzt man als Aktivator für Faktor II Faktor Xa unter Zusatz von Kephalin zu.

Diese bevorzugten Ausführungsformen der Erfindung berücksichtigen die Tatsache, daß die Konzentrationen der Gerinnungsparameter Faktoren XII, XI, IX, VIII, X, V und II die Zeit der Thrombinbildung beeinflussen derart, daß ein bestimmter Thrombinschwellenwert umso eher erreicht wird, je höher die Konzentration dieser Faktoren ist.

Die Thrombinbildung wird im Rahmen der Erfindung nach an sich bekannten Methoden bestimmt. Geeignet hierfür ist die partielle Thromboplastinzeit (PTT)-Methode. Bei ihrer Durchführung wird zweckmäßig ein Aktivator für Faktor XII zugesetzt. Die Thrombinbildung kann auch nach der Prothrombinzeit-Methode bestimmt werden. In diesem Falle setzt man zweckmäßig einen Aktivator für Faktor VII und F Va zu.

Als chromogenes Thrombinsubstrat kann im Rahmen der
Erfindung jedes für die Thrombinbestimmung geeignete
chromogene Substrat verwendet werden. Bevorzugt wird
jedoch im Rahmen der Erfindung H-D-Phe-Pip-Arg-pNA oder
Tos-Gly-Pro-Arg-pNA verwendet, wobei pNA = para-Nitroanilin bedeutet. Das pNA stellt den chromogenen Bestandteil der Substrate dar und wird durch das gebildete Thrombin abgespalten, so daß es photometrisch in
bekannter Weise bestimmt werden kann.

Als Gerinnungssystem kann beispielsweise eine Mischung
der Faktoren II bis XII oder Substratplasma (z. B. Normalcitratplasma) eingesetzt werden.

Im Rahmen der Erfindung kann jedes Plasma eingesetzt
werden, bevorzugt wird Citratplasma. Vor der Protein-C-
Bestimmung aus Plasma sollte eine Probenvorbereitung
durchgeführt werden. Hierzu kann jedes Adsorbens verwendet werden, welches Vitamin-K-abhängige (carboxylierte) Proteine binden kann (z. B. Bariumcitrat,
Aluminiumhydroxid).

Das Verfahren kann bei neutralen oder schwach alkalischen
pH-Werten durchgeführt werden, vorzugsweise zwischen pH
6 und 9. Als Puffer können die in diesem Bereich wirksamen
physiologisch unbedenklichen Puffer verwendet werden
wie z. B. Tris/HCl. Ferner können die für Gerinnungstests
üblichen Stabilisatoren und Konservierungsmittel wie
Rinderserumalbumin, Merthiolat und dergleichen, ebenfalls zugesetzt werden.

Die folgenden Beispiele erläutern die Erfindung weiter
in Verbindung mit der beigefügten Zeichnung. In dieser
stellen dar:

Fig. 1     eine Eichkurve für die Verlängerung der partiellen Thromboplastinzeit durch aktives Protein C aus Plasma (150 bis 0 %) und mit Tos-Gly-Pro-Arg-pNA (Chromozym®️ TH) als chromogenes Substrat.

Fig. 2     die Verlängerung der partiellen Thromboplastinzeit durch reines aktives Protein C mit Chromozym®️ TH als Substrat.

Fig. 3     die Verlängerung der Prothrombinzeit durch reines aktives Protein C mit Substrat Chromozym®️ TH.

Fig. 4     die Verringerung der Anfangssteigung durch reines aktives Protein C in einem chromogenen Prothrombintest. Substrat ebenfalls Chromozym®️ TH.

B e i s p i e l     1

Protein C-Bestimmung aus Plasma über Verlängerung der partiellen Thromboplastinzeit (Faktor VIII-Inaktivierung)

Reagentien:

Lösung 1: 50 mmol/l Natriumcitrat, 0,00025 % Polypren und 0,01 % Merthiolat,

Lösung 2: 250 mmol/l Bariumchlorid, 150 mmol/l Natriumchlorid und 0,01 % Merthiolat,

Lösung 3: 200 mmol/l MES, 150 mmol/l Natriumcitrat und 0,01 % Merthiolat, pH 6,0,

Lösung 4: 6 U/ml Thrombin in 100 mmol/l Tris/HCl,
pH 8,0, 0,1 % RSA und 0,01 % Merthiolat,

Lösung 5: 9 U/ml Antithrombin III und 0,25 USP/ml
Heparin in 100 mmol/l Tris/HCl, pH 8,
100 mmol/l NaCl, 2 % BSA, 1,5 % Saccharose
und 0,01 % Merthiolat,

Reagenzlösung: Kephalin und Ellagsäure als Faktor
XII-Aktivator in 10 mmol/l Tris/HCl,
pH 7,6,

Startreagenz: 1,1 mmol/l Chromozym ® TH = Tos-Gly-
Pro-Arg-pNA und 100 mmol/l Calciumacetat.

a) Proben

Normalcitratplasmapool: Als Spender kommen Personen
in Betracht, deren Quick-Wert zwischen 80 bis 120%
liegt. Die Eichkurve wird mit diesem Normalpool
erstellt. Der PC-Gehalt des Normalcitratplasmapools
wird 100 % gesetzt. Unterschiedliche Protein
C-Konzentrationen werden durch Verdünnen des Pools
mit Natriumcitrat (20 mmol/l) erhalten. Abstufungen:
150%, 100%, 75%, 50%, 25%, 10%, 0%. Für den 150%-Wert
wird statt 200µl 300µl Pool als Probe eingesetzt.

Drei Citratplasmen mit unterschiedlichem Protein
C-Antigengehalt werden als Kontrolle mitgeführt.

b) Probenvorbereitung

200 µl Probe werden mit 100 µl Lösung 1 10 Minuten
bei 25°C inkubiert. Nach Zugabe von 200 µl der
eiskalten (0°C) Lösung 2 wird 10 Minuten bei 0°C
inkubiert. Das Sediment wird 5 Minuten bei 4000
Umdrehungen pro Minute (Rotordurchmesser 17 cm)
und 0°C abzentrifugiert, mit 100 µl der 1:2 mit
Wasser verdünnten Lösung 2 bei 0°C gewaschen und erneut zentrifugiert. Das Sediment wird in 100 µl
Lösung 3 aufgenommen und 5 Minuten bei 25°C inkubiert, anschließend nochmals zentrifugiert. Der
Überstand = Eluat enthält neben anderen Vitamin
K-abhängigen Proteinen auch Protein C.

20 µl Eluat (Protein C) werden mit 100 µl Lösung 4
60 Minuten bei 37°C inkubiert. Protein C (PC) wird
dabei zu aktivem PC (APC) aktiviert. Zur Inaktivierung von Thrombin werden anschließend 100 µl
Lösung 5 zugemischt und die Mischung 60 Minuten
bei 37°C inkubiert. Die Mischung, die neben aktiviertem Protein C (APC) einen gegenüber Thrombin
50%igen Überschuß an Antithrombin III enthält,
wird in den chromogenen Gerinnungstest als Probe
eingesetzt.

c)  Messung der APC-Konzentration mittels Verlängerung
der partiellen Thromboplastinzeit
50 µl aktiviertes Protein C (Mischung aus b, siehe
oben), 100 µl Normalcitratplasma und 1000 µl Reagenzlösung werden in einer Mikroplastikküvette 5
Minuten bei 37°C inkubiert. Nach Zugabe von 100 µl
Startreagenz wird in einem Photometer bei 405 nm
und 37°C die Zeit bestimmt, bis eine definierte
Menge Substrat von neu entstandenem Thrombin zu
Tos-Gly-Pro-Arg-Peptid und para-Nitroanilin (=pNA)
umgesetzt ist. Die Menge umgesetzten Substrats,
die gemessen wird, wird durch einen Schwellenextinktionswert (z. B.$\Delta E=0,2$) definiert. Die Konzentration
der Gerinnungsparameter F XII, F XI, F IX, F VIII,
F X, F V und F II beeinflussen die Zeit, je höher
konzentriert sie sind, desto schneller wird der
Schwellenwert erreicht. Da APC die Faktoren VIII
und V proteolytisch inaktiviert, wird das endogene Gerinnungssystem (F XII - F II) empfindlich gestört und somit die partielle Thromboplastinzeit (PTT) verlängert. Unter den angegebenen Bedingungen nimmt die Verlängerung der partiellen Thromboplastinzeit proportional mit der Konzentration von APC (bzw. PC im Plasma) zu.

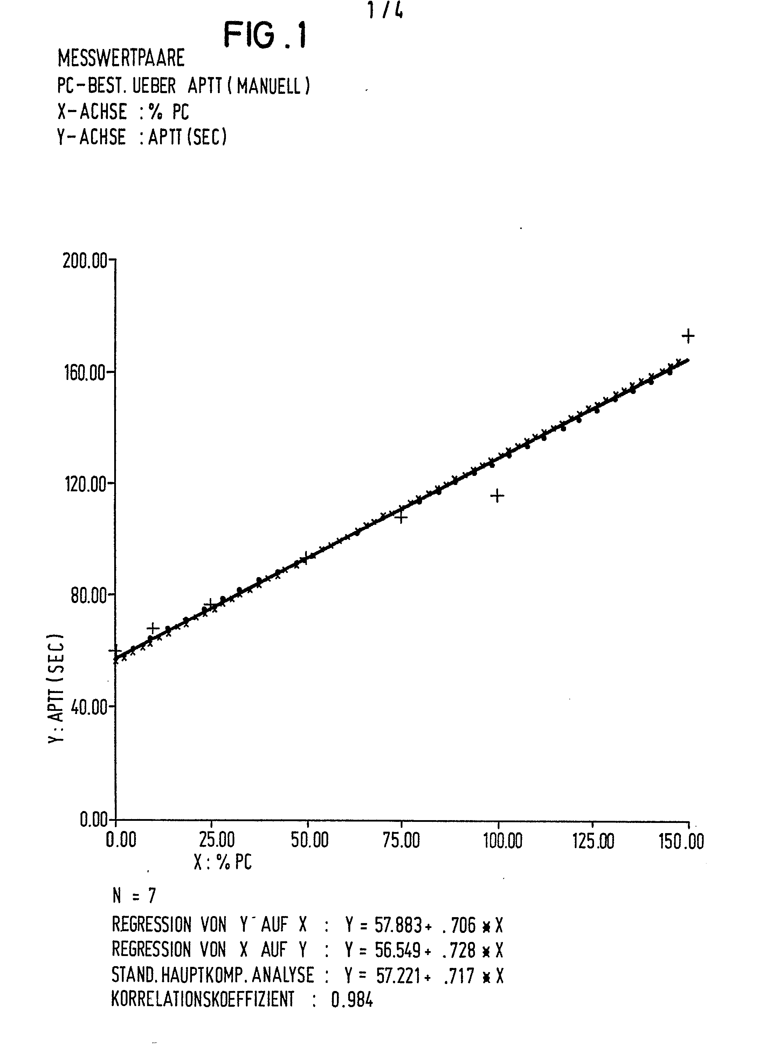

Zur Auswertung wird eine Eichkurve (Fig. 1) mit den Meßwertpaaren Plasma/Plasmaverdünnungen (150 bis 0%) und PTT-Zeiten erstellt. Für Plasmen unbekannter PC-Konzentration wird ebenfalls die PTT-Zeit ermittelt und aus der Eichkurve die entsprechende PC-Konzentration abgelesen.

d)  Ergebnisse

In Tabelle 1 sind die mit Plasmapool, Plasmapoolverdünnungen und Kontrollplasmen ermittelten PTT-Zeiten zusammengefaßt. Die PTT-Zeiten nehmen proportional zur PC-Konzentration in der Probe (0% bis 150%) zu. Die Protein C-Aktivitäten in den Kontrollplasmen entsprechen den Protein C-Antigenkonzentrationen, d. h. die Wiederfindung ist im Normalbereich (80 bis 120%) wie im Abnormalbereich (20 bis 40%) gut.

T a b e l l e    1

Verlängerung der PTT-Zeit durch APC aus Plasmaverdünnungen und Kontrollplasmen

| Probe | % Antigen | PTT (sec) | % Aktivität |
|---|---|---|---|
| Plasmaverdünnungen | 150 | 173,5 | 150 |
| | 100 | 116 | 100 |
| | 75 | 108 | 75 |
| | 50 | 93 | 50 |
| | 25 | 76,5 | 25 |
| | 10 | 68 | 10 |
| | 0 | 60 | 0 |
| Kontrollplasmen | 114 | 121 | 102 |
| | 81 | 115 | 84,5 |
| | 23,5 | 71 | 16 |

B e i s p i e l     2

Reines Protein C - Bestimmung mittels Verlängerung der
partiellen Thromboplastinzeit (Faktor VIII-Inaktivierung)
Die Zusammensetzung der Lösungen ist im Beispiel 1 angegeben.

a)     Vorbereitung
       Protein C wird aus Plasma nach der Methode von
       Comp et al., Blood **63** (1984) 15-21, gereinigt.
       20 µl reines Protein C in Puffer, bestehend aus
       100 mmol/l Tris/HCl, pH 8,0, 0,1% BSA und 0,01%
       Merthiolat, wird mit 100 µl Lösung 4 60 Minuten
       bei 37°C inkubiert. Thrombin wird dann durch
       Zugabe von 100 µl Lösung 5 und 60 Minuten Inkubation bei 37°C inaktiviert. Die Aktivität von
       aktiviertem Protein.C in dieser Mischung wird mit
       einem Substrat, Acetyl Pro-Pro-Arg-pNA (Hersteller: Pentapharm, Basel), dessen para-Nitroanilin
       von APC abgespalten wird, bestimmt (vgl. Lit.
       Bertina et al., Thromb. Haemostas. **51** (1) 1984,
       1-5) und in U/ml angegeben. 1 U/ml ist definiert
       als die Menge an aktiviertem Protein C, die nach
       oben beschriebener Probenvorbereitung eines Normalcitratplasmapools (100%) in der Mischung enthalten
       ist.

b)     Testdurchführung
       20 µl aktiviertes Protein C (Mischung aus 2a,
       siehe oben, unterschiedliche Konzentrationen
       werden durch Verdünnen mit Puffer, bestehend aus
       100 mmol/l Tris/HCl, pH 8,0, 0,1 % BSA und 0,01 %
       Merthiolat erhalten), 100 µl Plasma (Zusammensetzung: 5 Teile Faktor VIII-Mangelplasma und 1 Teil
       Normalcitratplasma) und 1000 µl Reagenzlösung

werden in einer Mikroplastikküvette 5 Minuten bei
37°C inkubiert. Nach Zugabe von 100 µl Startreagenz,
bestehend aus 1,1 µmol/l Chromozym TH = Tos-Gly-Pro-
Arg-pNA und 120 µmol/l Calciumlactat, wird in
einem Photometer bei 405 nm und 37°C die Zeit bestimmt, bis eine definierte Menge Substrat von neu
entstandenem Thrombin zu Tos-Gly-Pro-Arg-Peptid
und para-Nitroanilin umgesetzt ist. Die Menge
umgesetzten Substrats, die gemessen wird, wird
durch einen Schwellenextinktionswert (z. B. $\Delta E$ =
0,2) definiert.

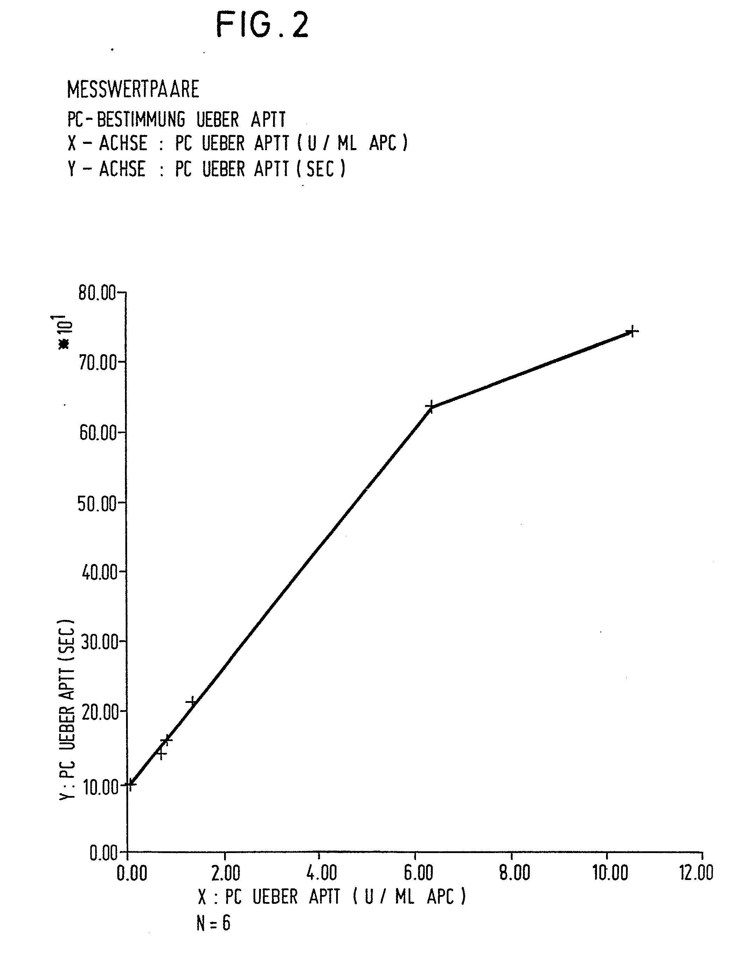

c)   Ergebnisse
Wird die Konzentration von aktiviertem Protein C
in der Mischung von 0 auf 10,6 U/ml variiert, so
verlängert sich die Zeit bis zum Erreichen des
Schwellenwertes (PTT-Zeit) von 96,6 auf 744 Sekunden (Fig. 2). Die Zunahme der PTT-Zeit ist bis zu
6,4 U/ml der APC-Konzentration direkt proportional.


B e i s p i e l    3


Protein C-Bestimmung mittels Verlängerung der Prothrombinzeit (Faktor V-Inaktivierung)


Reagentien:
Pufferlösung: 100 mmol/l Tris/HCl, pH 8,0 und 0,1 % BSA
             und 0,01 % Merthiolat
Reagenzlösung: Thromboplastin aus Kaninchenhirn als
             Faktor VII-Aktivator, 1,7 µmol/l Chromo-
             zym TH = Tos-Gly-Pro-Arg-pNA, 6 mmol/l
             $CaCl_2$ in 100 mmol/l Tris/HCl, pH 8,1.

a) Vorbereitung

Es wird ebenfalls reines Protein C mit Thrombin aktiviert, anschließend das Thrombin mit Antithrombin III/Heparin inaktiviert und als Probe eingesetzt (Beschreibung siehe 2a).

b) Testdurchführung

20 µl aktiviertes Protein C (Mischung aus 2a), 50 µl Plasma (Zusammensetzung 1 Teil Normalcitrat-plasma und 4 Teile 0,9%ige NaCl-Lösung) und 50 µl Faktor Va (1 U/ml in Puffer) werden in einer Mikroplastikküvette für 10 Minuten bei 37°C inkubiert. Nach Zugabe von 1000 µl Reagenzlösung wird in einem Photometer bei 405 nm und 37°C die Zeit bestimmt, bis eine definierte Menge Substrat von neu entstandenem Thrombin zu Tos-Gly-Pro-Arg-Peptid und para-Nitroanilin umgesetzt ist. Die Menge umgesetzten Substrats, die gemessen wird, wird durch einen Schwellenextinktionswert (z.B. $\Delta E=0,1$) definiert. Die Konzentration der Gerinnungsparameter F VII, F X, F V und F II beeinflussen die Zeit - je höher konzentriert sie sind, desto schneller wird der Schwellenwert erreicht. Da APC den Faktor V proteolytisch inaktiviert, wird das exogene Gerinnungssystem (F VII - F II) empfindlich gestört und somit die Prothrombinzeit (PT) verlängert.

c) Ergebnisse

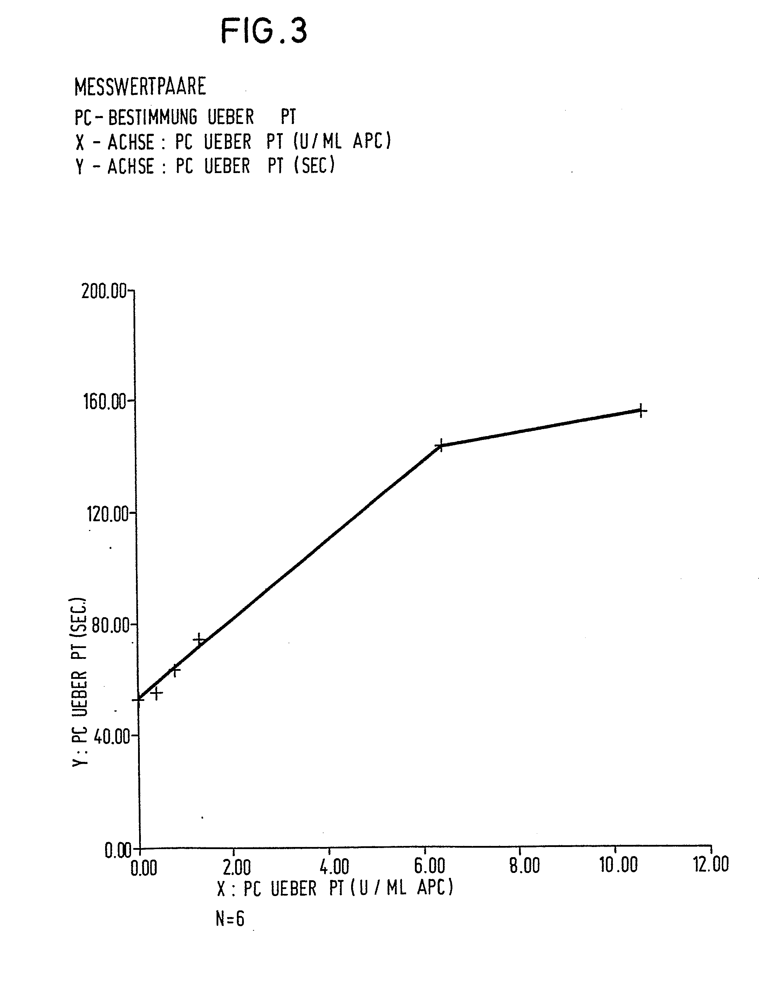

Wird die Konzentration von aktiviertem Protein C in der Mischung von 0 auf 10,6 U/ml variiert, so verlängert sich die Zeit bis zum Erreichen des Schwellenwertes (PT-Zeit) von 52,8 auf 156 Sekunden (Fig. 3). Die Zunahme der PT-Zeit ist bis zu 6,4 U/ml der APC-Konzentration direkt proportional.

B e i s p i e l   4

Protein C-Bestimmung mittels eines chromogenen Prothrombintests (Faktor V-Inaktivierung)

Reagenzien:

Prothrombinreagenzlösung:
75 mmol/l Tris/Imidazol/HCl, pH 8,4, 75 mmol/l NaCl,
50 mmol/l $CaCl_2$ und 0,005 mmol/l Kephalin.

a)   Vorbereitung
     Es wird ebenfalls reines Protein C mit Thrombin
     aktiviert, anschließend das Thrombin mit Antithrombin III/Heparin inaktiviert und als Probe
     eingesetzt (Beschreibung siehe 2a).

b)   Testdurchführung
     20 µl aktiviertes Protein C (Mischung aus 2a),
     50 µl Plasma (Zusammensetzung 1 Teil Normalcitratplasma und 60 Teile 0,9%ige NaCl-Lösung) als Prothrombin und Faktor V-Quelle und 1000 µl Prothrombinreagenz werden in einer Mikroplastikküvette 5
     Minuten bei 37°C inkubiert, nach Zugabe von 20 µl
     Faktor Xa (2 U/ml in einer 0,9%igen NaCl-Lösung)
     wird weitere 5 Minuten bei 37°C inkubiert. Die
     Farbreaktion wird mit Zugabe von 50 µl Substrat
     Chromozym ® TH (3,75 mmol/l) gestartet und bei 405
     nm und 37°C photometrisch verfolgt.

Das neu gebildete Thrombin setzt das Substrat zu Tos-Gly-Pro-Arg-Peptid und para-Nitroanilin um. Es wird die Extinktionsänderung pro Minute im Reaktionsanfang gemessen. Unter den beschriebenen Bedingungen beeinflussen die Konzentrationen der Gerinnungsfaktoren Xa, V und II die Extinktionsänderung pro Minute im Reaktionsanfang. Da APC den Faktor V proteolytisch inaktiviert, wird dieses System empfindlich gestört, d. h. die Umwandlung von Prothrombin zu Thrombin ist bis zum Meßzeitpunkt noch nicht vollständig abgelaufen und die Anfangssteigung der Thrombin-Substratreaktion wird umso kleiner, je mehr APC im Meßansatz vorhanden ist.

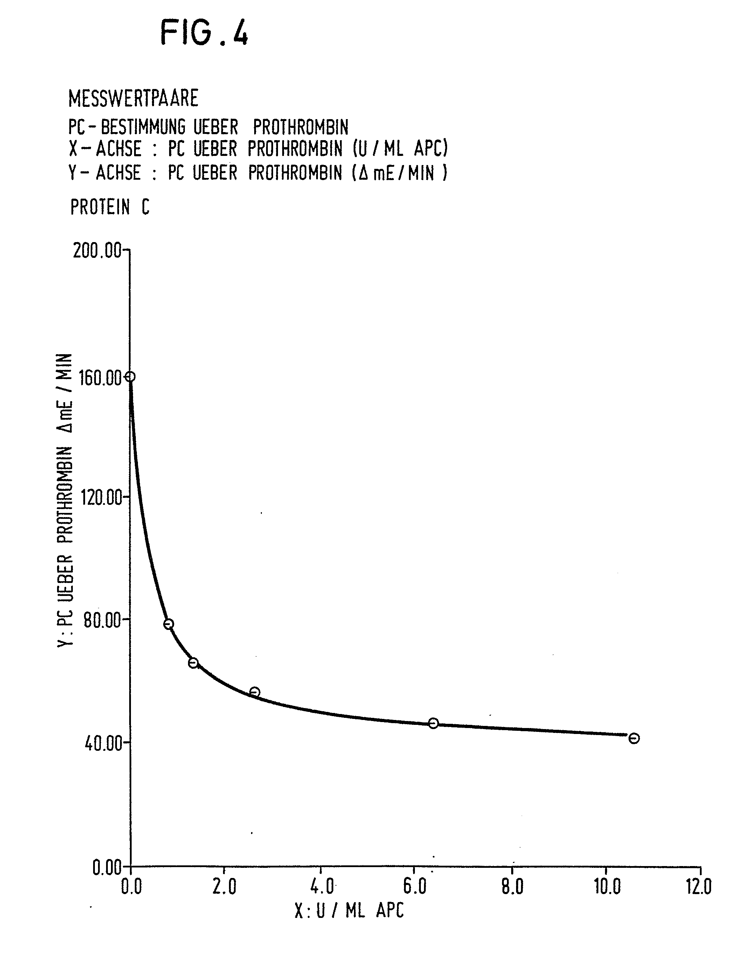

c)  Ergebnisse
Wird die Konzentration von aktiviertem Protein C in der Mischung von 0 auf 10,6 U/ml erhöht, verringert sich die Extinktionsänderung pro Minute im Meßanfang von 159 mE/min auf 42 mE/min (Fig. 4). Die Anfangssteigung nimmt mit der APC-Konzentration exponentiell ab.


B e i s p i e l    5

Einfluß von AT III-Überschuß in Gegenwart von Heparin auf die partielle Thromboplastinzeit - Einsatz zweier Thrombinsubstrate

a)  Testdurchführung
50 µl einer Mischung von AT III (Konzentration 0, 2,4, 3,6, 4,9, 6,1 U/ml) und Heparin als Cofaktor (Konzentration 0,12 USP/ml), 100 µl Normalcitratplasma und 1000 µl Reagenzlösung gemäß Beispiel 1 werden in einer Mikroplastikküvette 5 Minuten bei 37°C inkubiert. Nach Zugabe von 100 µl Startreagenz, bestehend aus 1,1 µmol/l Substrat (Chromozym ® TH = Tos-Gly-Pro-Arg-pNA oder S 2238 = H-D-Phe-Pip-Arg-pNA) und 120 µl Calciumacetat, wird in einem Photometer bei 405 nm und 37°C die Zeit bestimmt, bis eine definierte Menge Substrat von entstandenem Thrombin zum Peptid und para-Nitroanilin umgesetzt ist. Die Menge umgesetzten Substrats, die gemessen wird, wird durch einen Schwellenextinktionswert (z. B. $\Delta E = 0,2$) definiert.

b)   Ergebnisse

In Tabelle 2 sind die in Abwesenheit und in Gegenwart von AT III ermittelten PTT-Zeiten für beide Substrate zusammengefaßt. Mit beiden Thrombinsubstraten werden die PTT-Zeiten durch AT III-Zusatz nicht verlängert, d. h. neu entstandenes Thrombin reagiert auch bei AT III-Überschuß unter den gegebenen Reaktionsbedingungen zunächst mit dem Substrat.

<u>T a b e l l e   2</u>

AT III-Einfluß auf die PTT-Substrate: Chromozym ® TH
und S 2238

| Heparin USP/ml | AT III U/ml | PTT (sec) Chromozym ® TH | PTT (sec) S 2238 |
|---|---|---|---|
| 0,12 | 0 | 75 | 72,5 |
| | 2,4 | 72,5 | 72,5 |
| | 3,6 | 72 | 72 |
| | 4,9 | 72 | 71 |
| | 6,1 | 72 | 73 |

<u>P a t e n t a n s p r ü c h e</u>

1. Verfahren zur photometrischen Bestimmung von Protein C, insbesondere im Plasma, d a d u r c h    g e k e n n z e i c h n e t , daß die das zu bestimmende Protein C enthaltende Probe mit Thrombin unter Bildung von aktiviertem Protein C inkubiert wird, danach überschüssiger Thrombininhibitor zugesetzt wird und anschließend die Abnahme der durch Gerinnungsfaktoren vermittelten Bildung von Thrombin aus Prothrombin mittels eines chromogenen Thrombinsubstrates bestimmt wird.

2. Verfahren nach Anspruch 1,    d a d u r c h g e k e n n z e i c h n e t ,    daß man als Thrombininhibitor Antithrombin III gegebenenfalls unter Zusatz von Heparin verwendet.

3. Verfahren nach Anspruch 1 oder 2, d a d u r c h    g e k e n n z e i c h n e t , daß man als chromogenes Thrombinsubstrat H-D-Phe-Pip-Arg-pNA oder Tos-Gly-Pro-Arg-pNA verwendet.

4. Verfahren nach einem der Ansprüche 1 bis 3, d a d u r c h    g e k e n n z e i c h n e t , daß man einen Aktivator für Faktor XII zusetzt.

5. Verfahren nach Anspruch 4,    d a d u r c h g e k e n n z e i c h n e t ,    daß man Kephalin und Ellagsäure zusetzt.

6.  Verfahren nach Anspruch 1 bis 3,
    d a d u r c h    g e k e n n z e i c h n e t ,
    daß man einen Aktivator für Faktor VII zusetzt.

7.  Verfahren nach Anspruch 6,    d a d u r c h
    g e k e n n z e i c h n e t ,    daß man
    Faktor V sowie als Faktor VII-Aktivator Thromboplastin zusetzt.

8.  Verfahren nach einem der Ansprüche 1 bis 3,
    d a d u r c h    g e k e n n z e i c h n e t ,
    daß man einen Aktivator für Faktor II zusetzt.

9.  Verfahren nach Anspruch 8,    d a d u r c h
    g e k e n n z e i c h n e t ,    daß man
    Kephalin und Faktor Xa zusetzt.

## FIG.1

MESSWERTPAARE
PC-BEST. UEBER APTT (MANUELL)
X-ACHSE : % PC
Y-ACHSE : APTT (SEC)

N = 7

REGRESSION VON Y AUF X  :  Y = 57.883 + .706 ✳ X
REGRESSION VON X AUF Y  :  Y = 56.549 + .728 ✳ X
STAND. HAUPTKOMP. ANALYSE :  Y = 57.221 + .717 ✳ X
KORRELATIONSKOEFFIZIENT  :  0.984

# FIG.2

MESSWERTPAARE

PC-BESTIMMUNG UEBER APTT
X - ACHSE : PC UEBER APTT ( U / ML APC )
Y - ACHSE : PC UEBER APTT ( SEC )

X : PC UEBER APTT ( U / ML APC )
N = 6

# FIG.3

MESSWERTPAARE

PC - BESTIMMUNG UEBER    PT
X - ACHSE : PC UEBER  PT (U / ML APC)
Y - ACHSE : PC UEBER  PT (SEC)

N=6

## FIG.4

MESSWERTPAARE

PC-BESTIMMUNG UEBER PROTHROMBIN
X-ACHSE : PC UEBER PROTHROMBIN (U / ML APC)
Y-ACHSE : PC UEBER PROTHROMBIN (Δ mE / MIN )

PROTEIN C

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,D | THROMBOSIS & HAEMOSTASIS, Band 51, Nr. 1, 1984, Seiten 1-5, Stuttgart; R.M. BERTINA et al.: "The use of a functional and immunologic assay for plasma protein C in the study of the heterogeneity of congenital protein C deficiency" * Seite 2, Spalte 1, Zeilen 32-47, Abbildung 1 * | 1,2 | C 12 Q 1/56<br>C 12 Q 1/38 //<br>G 01 N 33/68 |
| | --- | | |
| X | CHEMICAL ABSTRACTS, Band 100, Nr. 19, 7. Mai 1984, Seite 216, Spalten 1-2, Zusammenfassungsnr. 152824t, Columbus, Ohio, US; N. SALA et al.: "A functional assay of protein C in human plasma", & BLOOD 1984, 63(3), 671-675 | 1,2 | |
| | --- | | **RECHERCHIERTE SACHGEBIETE (Int. Cl.4)** |
| A | CHEMICAL ABSTRACTS, Band 97, Nr. 5, 2. August 1982, Seite 375, Spalte 2, Zusammenfassungsnr. 36731f, Columbus, Ohio, US; R.A. MARLAR et al.: "Mechanism of action of human activated protein C, a thrombin-dependent anticoagulant enzyme", & BLOOD 1982, 59(5), 1067-1072 | 1 | C 12 Q 1/00<br>G 01 N 33/00 |
| | --- | | |
| A | US-A-4 214 049 (Bo T. AF EKENSTAM) | | |
| | ---     -/- | | |

Der vorliegende Recherchenbericht wurde für alle Patentansprüche erstellt.

| Recherchenort | Abschlußdatum der Recherche | Prüfer |
|---|---|---|
| BERLIN | 23-02-1987 | GREEN C.H. |

Europäisches
Patentamt

**EUROPÄISCHER RECHERCHENBERICHT**

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CLINICAL CHEMISTRY, Band 29, Nr. 2, Februar 1983, Seiten 225-236, Washington DC, US; J. FAREED et al.: "Diagnostic efficacy of newer synthetic-substrates methods for assessing coagulation variables: A critical overview"<br>* Seite 226, Spalte 1, Zeilen 19-22 * | 3 | |
| X,P | EP-A-0 182 929 (AMERICAN HOSPITAL SUPPLY CORP.)<br>* Seiten 8/9, Beispiele 4, 5 * | 1-3 | |

RECHERCHIERTE
SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| BERLIN | 23-02-1987 | GREEN C.H. |